(19) **Europäisches Patentamt**
**European Patent Office**
**Office européen des brevets**

(11) **EP 2 163 267 B1**

(12) **EUROPEAN PATENT SPECIFICATION**

(45) Date of publication and mention
of the grant of the patent:
**06.11.2013 Bulletin 2013/45**

(51) Int Cl.:
***A61L 15/58*** *(2006.01)*

(21) Application number: **09252171.5**

(22) Date of filing: **11.09.2009**

(54) **Patch and patch preparation**

Flicken und Flickenherstellung

Patch et préparation de patch

(84) Designated Contracting States:
**AT BE BG CH CY CZ DE DK EE ES FI FR GB GR HR HU IE IS IT LI LT LU LV MC MK MT NL NO PL PT RO SE SI SK SM TR**

(30) Priority: **12.09.2008 JP 2008234604**

(43) Date of publication of application:
**17.03.2010 Bulletin 2010/11**

(73) Proprietor: **Nitto Denko Corporation**
**Ibaraki-shi**
**Osaka 567-8680 (JP)**

(72) Inventors:
• **Funakoshi, Yoshio**
**Ibaraki-shi,**
**Osaka 567-8680 (JP)**

• **Kasahara, Tsuyoshi**
**Ibaraki-shi,**
**Osaka 567-8680 (JP)**
• **Hamada, Atsushi**
**Ibaraki-shi,**
**Osaka 567-8680 (JP)**
• **Ishikura, Jun**
**Ibaraki-shi,**
**Osaka 567-8680 (JP)**

(74) Representative: **Duckworth, Timothy John**
**J A Kemp**
**14 South Square**
**Gray's Inn**
**London WC1R 5JJ (GB)**

(56) References cited:
**EP-A2- 2 011 525      EP-A2- 2 011 526**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

**Description**

TECHNICAL FIELD OF THE INVENTION

The present invention relates to a patch and a patch preparation showing appropriate skin adhesiveness and flexibility, superior adhesion to the skin, and less adhesive residue upon peeling from the skin, which can be preferably used for protection of a skin lesion and a wounded surface, transdermal absorption of a medicament and the like.

BACKGROUND OF THE INVENTION

Conventionally, patch and patch preparation for the skin and the like have been used for the protection of a skin lesion and a wounded surface, transdermal absorption of a medicament and the like. Such patch and patch preparation generally have a constitution wherein a layer made of an adhesive composition containing a medicament as necessary, namely, an adhesive layer, is formed on at least one surface of a flexible support. The aforementioned adhesive layer is generally requested to have the following properties.

(1) skin adhesion: Patch and the like need to maintain an adhesion state without being detached from the skin surface for a given period.
(2) removability: While an adhesive layer is required to provide the above-mentioned skin adhesion, when it is not easily removed from the skin after use due to too high a skin adhesion strength thereof, peeling causes pain, and the adhesive layer remains on the skin surface to cause adhesive residue.
(3) low irritation: When an adhesive layer is strongly irritative to the skin, skin rash is induced.
(4) followability to skin deformation: For sufficient protection of a skin lesion and a wounded surface, as well as transdermal absorption of medicaments, an adhesive layer should be able to sufficiently follow skin deformation caused by physical movement of the body, so that patch and the like will not lose adhesiveness due to skin deformation.

Thus, an adhesive layer to be used for patch and the like is required to show suitable skin adhesion, removability after lapse of given time and followability to skin deformation. As a composition to form such adhesive layer, various gel compositions such as those comprised of hydrogel compositions, acrylic gel compositions and the like are used.

Of the above-mentioned gel compositions, hydrogel compositions containing water do not stand desiccation and lack stability, since they tend to become hard when water is lost over time. The hardened gel compositions are insufficient in adhesion to the skin and followability to skin deformation. On the other hand, as a gel composition free of water, an acrylic gel composition wherein an organic liquid component is held by crosslinked acrylic adhesive is disclosed (JP-A-3-220/20). However, since an adhesive layer having a certain level of thickness is difficult to form on a support using the aforementioned acrylic gel composition, the layer has a limited ability to retain or absorb a medicament, an organic liquid component such as a transdermal absorption promoter and the like, and the like, and therefore, cannot hold them in large amounts. Since an adhesive layer having a large thickness is difficult to form using the above-mentioned compositions, when a patch and the like containing the above-mentioned compositions as an adhesive layer is adhered to the skin, an improvement in the ability to absorb external impacts and the like to protect the skin is necessary.

Moreover, a gel composition wherein a synthetic rubber polymer is crosslinked is known, and a gel composition containing a rubber component containing a rubber having a functional group and a liquid oil compatible with the rubber component in a proportion of 20 parts by weight - 80 parts by weight of the liquid oil per 100 parts by weight of the rubber component, which are crosslinked therein, is disclosed (JP-A-10-151185).

In the above-mentioned JP-A-10-151185, however, a patch or patch preparation having an adhesive layer having a gel fraction in a given range mentioned below, which is comprised of a composition containing 33 wt% - 65 wt% of a rubber component having a functional group, is not disclosed.

[SUMMARY OF THE INVENTION]

Accordingly, the present invention aims to provide a patch having an adhesive layer with a thickness sufficient for protection of a skin lesion and a wounded surface, which is flexible, adheres well to the skin, provides good feeling during adhesion, leaves less adhesive residue when removed from the skin, and shows good followability to skin deformation, as well as a patch preparation capable of sufficiently retaining a medicament and the like.

Means of Solving the Problems

The present inventors have conducted intensive studies in an attempt to solve the aforementioned problems and found that the above-mentioned problems of the present invention can be solved by forming an adhesive layer having a gel fraction in a given range, which is comprised of a composition containing 33 wt% - 65 wt% of a liquid rubber having a crosslinkable functional group in a molecule, on at least one surface of a support, which resulted in the completion of the present invention.

Accordingly, the present invention provides the following [1] - [9].

[1] A patch comprising a support and an adhesive layer formed on at least one surface of the support, wherein the adhesive layer has a gel fraction of 50 wt% - 75 wt%, and is comprised of a composition containing 33 wt% - 65 wt% of a liquid rubber having 3-20 crosslinkable functional groups in a molecule, wherein the composition is crosslinked using a crosslinking agent in 0.01 wt% to 4 wt% of the total amount of the composition used for preparation of the adhesive layer, and the gel fraction means the ratio of the weight of insoluble matter obtained by immersing an adhesive layer in toluene, to the components involved in the crosslinking of the adhesive layer.

[2] The patch of the above-mentioned [1], wherein the adhesive layer is comprised of a composition further containing 10 wt% - 40 wt% of an organic liquid component.

[3] The patch of the above-mentioned [1] or [2], wherein the liquid rubber is a liquid isoprene rubber having a crosslinkable functional group in a molecule.

[4] The patch of the above-mentioned [2] or [3], wherein the organic liquid component is one or more kinds selected from fats and oils, hydrocarbon, fatty acid ester and higher fatty acid.

[5] The patch of the above-mentioned [4], wherein the fatty acid ester is fatty acid monoalkyl ester.

[6] The patch of the above-mentioned [5], wherein the fatty acid monoalkyl ester is one or two kinds selected from isopropyl myristate and isopropyl palmitate.

[7] The patch of any one of the above-mentioned [1] - [6], wherein the adhesive layer is comprised of a composition further containing 15 wt% - 45 wt% of a tackifier.

[8] The patch of any one of the above-mentioned [1] - [7], wherein the adhesive layer has a thickness of not less than 40 $\mu$m.

[9] A patch preparation comprising the patch of any one of the above-mentioned [1] - [8], wherein the adhesive layer contains a medicament.

Effect of the Invention

According to the present invention, a patch having an adhesive layer having a sufficient thickness and superior in the protection of a skin lesion and a wounded surface, as well as a patch preparation capable of retaining a sufficient amount of a medicament or an organic liquid component such as percutaneous absorption promoter etc. can be provided. Since the patch and patch preparation of the present invention have appropriate adhesiveness and flexibility, they provide good adhesion to the skin, leave less adhesive residue when removed from the skin, and show good followability to skin deformation

[Embodiment of the Invention]

In the present invention, the above-mentioned liquid rubber having a crosslinkable functional group in a molecule forms a net-like structure upon crosslinking of the composition containing the liquid rubber, and confers flexibility and shape retainability to the adhesive layer. The liquid rubber is not particularly limited as long as it has a crosslinkable functional group in a molecule, and liquid isoprene rubber containing the aforementioned functional group, liquid butadiene rubber containing the aforementioned functional group, liquid isobutylene rubber containing the aforementioned functional group and the like can be used. One or more kinds of liquid rubbers can be used. Such liquid rubber is liquid even though it has a high molecular weight, and enables an adhesive layer to contain a large amount of organic liquid component with ease. The aforementioned liquid isoprene rubber containing a functional group is preferably used, since it can easily confer flexibility to an adhesive layer. In the present specification, the "liquid" means flowability at 25°C. The presence or absence of flowability can be evaluated by filling a sample in a container and evaluating deformation upon tilting the container.

The weight average molecular weight of a liquid rubber having a crosslinkable functional group in a molecule to be used in the present invention is not particularly limited as long as the rubber component is liquid. It is preferably 1,000 - 60,000, more preferably 10,000 - 40,000, most preferably 20,000 - 30,000. When the weight average molecular weight is less than 1,000, the flexibility of the adhesive layer may decrease, and a large amount of an organic liquid component may not be easily contained in an adhesive layer. On the other hand, when the weight average molecular weight exceeds 60,000, the rubber may not be liquid, the content uniformity of the adhesive layer may be difficult to maintain, and the compatibility of the aforementioned liquid rubber with other components contained in an adhesive layer may be difficult to secure.

Here, the "weight average molecular weight" is measured by gel filtration chromatography under the following conditions.

(analysis conditions)

Gel filtration chromatography apparatus: HLC8120 (manufactured by Tosoh Corporation)
column: TSK gel GMH-H(S) (manufactured by Tosoh Corporation) standard: polystyrene
eluent: tetrahydrofuran
flow rate: 0.5 mL/min
measurement temperature: 40°C
detector: differential refractometer

In the patch and patch preparation of the present invention, the composition used for the preparation of the adhesive layer contains a liquid rubber having a crosslinkable functional group in a molecule in an proportion of 33 wt% - 65 wt%, preferably 40 wt% - 60 wt%, of the total weight of the  composition. When the amount of the aforementioned liquid rubber is lower than 33 wt%, the adhesive layer thus formed may not have a sufficient strength. On the other hand, when the amount of the aforementioned liquid rubber exceeds 65 wt%, the adhesive layer thus formed may have decreased flexibility and may fall from the skin.

When the crosslinkable functional group in the above-mentioned liquid rubber is not particularly limited, amino group, carboxyl group, hydroxyl group, chlorosulfone group, ester group, epoxy group, isocyanate group, methylol group, sulfone group, mercapto group and the like may be used. The functional group may be used alone or in a combination of two or more kinds thereof. In consideration of formation of crosslinking with an epoxy compound as the below-mentioned crosslinking agent, carboxyl group and hydroxyl group are preferable.

When the number of crosslinkable functional groups in a molecule of the above-mentioned liquid rubber is not less than 3 functional groups, the aforementioned liquid rubber generally forms a net-like structure efficiently, and the effect of the invention can be sufficiently afforded. In view of the above, the number of crosslinkable functional groups in a molecule of the aforementioned liquid rubber is not less than 3, preferably not less than 5, more preferably not less than 8, further more preferably not less than 10.

In the present invention, since the above-mentioned liquid rubber has not less than 3 crosslinkable functional groups in a molecule, a three-dimensional net-like structure can be formed even without introducing a branching agent and the like into the main chain of the liquid rubber. Therefore, a large amount of an organic liquid component can be contained  in the adhesive layer.

On the other hand, when the number of the aforementioned functional groups in a molecule is too many, the amount of the necessary crosslinking agent increases. Thus, the number of the crosslinkable functional groups in a molecule is not more than 20, more preferably not more than 15, further preferably not more than 12. When the number of the functional groups in a molecule is too many, the remaining active groups increase in number, possibly affecting the components contained in an adhesive layer such as medicaments and the like, and the flexibility of an adhesive layer may decrease, resulting in poor followability to skin deformation.

The number of crosslinkable functional groups in a molecule of liquid rubber is measured as follows. That is, the total number of crosslinkable functional groups in a molecule of liquid rubber in a sample is determined, and the number is divided by the number average molecular weight of the liquid rubber to give the number of crosslinkable functional groups per one molecule of liquid rubber. The number of such functional groups can be determined according to the method described in the Japanese Industrial Standards (JIS) K0070 and the like, and the like. For example, when the functional group is a carboxyl group, the number of carboxyl groups in a sample is determined by an acid number measurement method using potassium hydroxide. When it is an ester group, the number can be determined by an ester number measurement method using potassium hydroxide, and when it is a hydroxyl group, the number can be determined by a hydroxyl group number measurement method including acetylation of a sample and neutralization thereof with potassium hydroxide. Here, the number average molecular weight of liquid rubber means a value obtained under the same conditions as the aforementioned weight average molecular weight.

In the present invention, an adhesive layer formed on at least one surface of a support characteristically has a gel fraction of 50 wt% - 75 wt%. When the gel fraction is lower than 50 wt%, gel strength of an adhesive layer decreases, due to which the layer may leave an adhesive residue when it is removed from the skin. On the contrary, when the gel fraction exceeds 75 wt%, the adhesive layer becomes hard, skin adhesion strength decreases and the soft feeling during adhesion may not be available.

In the present invention the "gel fraction" means the ratio of the weight of insoluble matter obtained by immersing an adhesive layer in toluene, to the components involved in the crosslinking of the adhesive layer. The gel fraction can be determined by immersing a sample of the composition for forming an adhesive layer in toluene at ambient temperature (23°C) for 7 days and applying the weight of the obtained insoluble matter to the following equation.

$$\texttt{gel fraction (wt\%)} = W_2/(W_1 \times A/B) \times 100$$

A: weight of liquid rubber having crosslinkable functional group in a molecule and crosslinking agent
B: total weight of adhesive layer
$W_1$: weight of sample taken from adhesive layer
$W_2$: weight of insoluble matter obtained by immersing, in toluene, sample taken from adhesive layer

The above-mentioned 50 wt% - 75 wt% of gel fraction in the present invention can be preferably achieved by applying an adhesive layer to a crosslinking treatment. In the present invention, a crosslinking treatment to be applied is not particularly limited and, for example, a physical crosslinking treatment using ultraviolet light irradiation, radiation irradiation such as electron beam irradiation and the like, or a chemical crosslinking treatment using various crosslinking agents is performed.

To prevent an adverse influence on components contained in an adhesive layer such as medicaments and the like, the composition is preferably crosslinked by adding a crosslinking agent to the composition used for preparation of the adhesive layer. Examples of the crosslinking agent include compounds such as amine compounds (e.g., diaminohexane and the like), bifunctional compounds (e.g., polyethylene glycol, polypropylene glycol, hexanediol, phthalic acid and the like), and tetrafunctional epoxy compounds (e.g., 1,3-bis(N,N-diglycidyl diaminomethyl)cyclohexane, 1,3-bis(N, N-diglycidyl diaminomethyl)benzene and the like); isocyanate compounds such as aromatic polyisocyanates (e.g., trilene diisocyanate, diphenylmethane diisocyanate, xylylene diisocyanate and the like), aliphatic polyisocyanates (e.g., hexamethylene diisocyanate and the like), alicyclic polyisocyanates (e.g., isophorone diisocyanate, hydrogenated diphenylmethane diisocyanate and the like), and the like; organic peroxides such as peroxyketal, dialkyl peroxide, diacyl peroxide and the like; organic metal salts such as acetylacetone aluminum salt, acetylacetone vanadium salt, acetylacetone molybdenum salt and the like; metal alcoholates such as tetrabutyl titanate and the like; metal chelate compounds such as aluminum glycinate, aluminum hydroxide gel and the like; acid chlorides such as adipyl dichloride and the like; monomers for multifunctional internal crosslinking such as diallylurea, ethylene glycol diacrylate, trimethylol propane triacrylate, ethylene glycol dimethacrylate, methylenebis(acrylamide), triallylamine and the like; polymers for multifunctional crosslinking such as polyamines (e.g., diallylamine hydrochloride polymer, methyl diallylamine hydrochloride polymer and the like), and polyallylamines (e.g., allylamine hydrochloride polymer, allylamine amide sulfate polymer and the like); and the like. The crosslinking agent may be used alone, or in a combination of two or more kinds thereof. When the crosslinkable functional group in a molecule of liquid rubber is a carboxyl group, an epoxy compound is preferably used from among the aforementioned crosslinking agents. This is because an epoxy group reacts with a carboxyl group in a molecule of liquid rubber and develops a hydroxyl group, and the hydroxyl group can react with other carboxyl group, whereby a net-like structure can be advantageously formed.

The amount of the above-mentioned crosslinking agent is 0.01 wt% - 4 wt%, more preferably 0.05 wt% - 2 wt%, of the total amount of the composition used for preparation of the adhesive layer.

In the patch and patch preparation of the present invention, the adhesive layer can contain an organic liquid component. The organic liquid component is combined with a net-like structure formed by crosslinking of the above-mentioned liquid rubber, and enhances the flexibility of gel in the adhesive layer. When a chemical substance such as medicaments and the like is contained in the adhesive layer, the organic liquid component dissolves or disperses the substance. The organic liquid component to be used in the present invention needs to have compatibility with the above-mentioned liquid rubber in the adhesive layer. An organic liquid component which is liquid and nonvolatile at ambient temperature is preferable. In the present invention, fats and oils such as olive oil, castor oil, lanolin and the like, hydrocarbon such as squalene and liquid paraffin, fatty acid ester such as fatty acid alkyl ester and the like, higher fatty acid such as oleic acid, caprylic acid and the like are preferable. The organic liquid component may be used alone, or in a mixture of two or more kinds thereof.

Particularly, as in the patch preparation of the present invention, when a medicament is contained in an adhesive composition, fatty acid ester is preferably used to promote transdermal absorption of the medicament. Examples of the fatty acid ester include fatty acid dialkyl ester such as dioctyl phthalate, diisopropyl adipate, diethyl sebacate and the like, fatty acid monoalkyl ester and the like. To enhance good transdermal absorption of a medicament, fatty acid monoalkyl ester is preferable.

When the carbon number of fatty acid constituting fatty acid monoalkyl ester is greater or smaller than necessary, compatibility with the above-mentioned liquid rubber may be degraded or fatty acid monoalkyl ester may be volatilized in the heating step during formulation of a preparation.

In the present invention, therefore, fatty acid monoalkyl ester comprised of saturated or unsaturated higher fatty acid having a carbon number of 12 - 16, more preferably 12 - 14, and saturated or unsaturated lower monovalent alcohol having a carbon number of 1 - 4 is preferably employed. For preservation stability, fatty acid monoalkyl ester comprised of saturated higher fatty acid and saturated lower monovalent alcohol is preferably used since it is free of oxidative decomposition and the like. Examples of the aforementioned higher fatty acid include lauric acid (C12), myristic acid (C14) and palmitic acid (C16), and myristic acid and palmitic acid are particularly preferable. Examples of the aforementioned lower monovalent alcohol include methyl alcohol, ethyl alcohol, propyl alcohol and butyl alcohol. They are not limited to straight chain alcohol and may be a branched chain alcohol. Particularly preferred is isopropyl alcohol. Most preferable fatty acid monoalkyl esters usable in the present invention are isopropyl myristate and isopropyl palmitate.

The aforementioned organic liquid component is preferably contained in a proportion of 10 wt% - 40 wt%, more preferably 15 wt% - 30 wt%, of the total amount of the composition to be used for preparation of an adhesive layer. When the content of the organic liquid component is less than 10 wt% of the total amount of the composition, the gel obtained after crosslinking reaction becomes too hard, thus possibly degrading the adhesiveness of the adhesive layer to the skin or followability to the skin. In addition, diffusional mobility and absorption of other components such as medicaments etc. in the adhesive layer may be degraded. On the other hand, when the content of the organic liquid component exceeds 40 wt% of the total amount of the composition, the gel strength of the adhesive layer may decrease to cause adhesive residue.

The patch and patch preparation of the present invention may further contain a general tackifier during preparation of an adhesive layer. Examples of the tackifier include naturally occurring substance and a derivative thereof such as rosin resin, terpene resin and the like, synthetic resins such as aliphatic petroleum resin, alicyclic petroleum resins such as alicyclic saturated hydrocarbon resin, petroleum resin, aromatic petroleum resin, coumarone-indene resin, styrene resin, phenol resin, xylene resin and the like, and the like. These tackifiers are preferably contained in a proportion of 15 wt% - 45 wt%, further preferably 15 wt% - 30 wt%, of the total amount of the composition to be used for preparation of an adhesive layer. When it is less than 15 wt%, the adhesive layer may not have sufficient adhesiveness, and when it is greater than 45 wt%, the tackifier may be separated in the adhesive layer to cause stickiness of the adhesive layer and/or leave adhesive residue when the adhesive layer is removed from the skin.

In the patch and patch preparation of the present invention, the adhesive layer may contain other optional components within the range that does not prevent the effect of the invention. For example, antioxidants such as ascorbic acid, tocopherol acetate, natural vitamin E, dibutylhydroxytoluene, butylhydroxyanisole and the like, amine-ketone anti-aging agents such as 2,6-tert-butyl-4-methylphenol and the like, aromatic secondary amine anti-aging agents such as N,N'-di-2-naphthyl-p-phenylenediamine and the like, monophenol anti-aging agents such as 2,2,4-trimethyl-1,2-dihydroquinoline polymer and the like, bisphenol anti-aging agents such as 2,2'-methylene-bis(4-ethyl-6-tertbutylphenol) and the like, polyphenol anti-aging agents such as 2,5-tert-butylhydroquinone and the like, fillers such as kaolin, hydrated silicon dioxide, zinc oxide, acrylic acid starch 1000 and the like, softening agents such as propylene glycol, polybutene, macrogol 1500 and the like, preservatives such as benzoic acid, sodium benzoate, chlorhexidine hydrochloride, sorbic acid, methyl p-hydroxybenzoate, butyl p-hydroxybenzoate and the like, colorants such as yellow iron oxide, yellow iron sesquioxide, iron sesquioxide, black iron oxide, carbon black, carmine, $\beta$-carotene, copper chlorophyll, Food Color blue No. 1, Food Color yellow No. 4, Food Color Red No. 2, licorice extract and the like, algefacients such as fennel oil, d-camphor, dl-camphor, peppermint oil, d-borneol, 1-menthol and the like, flavors such as spearmint oil, clove oil, vanillin, bergamot oil, lavender oil and the like, and the like can be contained.

The patch and patch preparation of the present invention can be produced generally as follows. That is, the above-mentioned liquid rubber and an organic liquid component are mixed in the presence of a solvent where necessary, a crosslinking agent is added thereto, the viscosity is adjusted where necessary with an organic solvent, and the mixture is applied to at least one surface of a support. Then, the solvent is evaporated at a suitable temperature to form crosslinking in the liquid rubber, thereby providing an adhesive layer on the support. Where necessary, the adhesive layer is aged. Furthermore, a release liner described below can also be press-bonded to give a laminate. In addition, an adhesive layer may be formed on the release liner, and a support may be press-bonded to an adhesive face of the adhesive layer.

While the support to be used in the present invention is not particularly limited, a support that does not substantially allow permeation of components contained in the adhesive layer, namely, a support that does not allow components contained in the adhesive layer to pass through the support and get lost from the back face of the support is preferable. As the above-mentioned support, a flexible material is preferably used since it is applied as a patch to the skin. Examples of the material include a film or sheet made of a synthetic resin such as plasticized flexible polyvinyl chloride, nonplasticized flexible polyvinyl chloride, polyethylene, polypropylene, polybutene, ethylene-methylmethacrylate copolymer, ethylene-vinyl acetate copolymer, ethylene-propylene rubber, polyamide, polyethylene terephthalate, polyurethane, polyvinyldene chloride, polyvinyl alcohol and the like. In addition, flexible films and sheets of non-woven fabric, foamed plastic sheet, cellulose, acetyl cellulose and the like can also be used. Moreover, a release liner made of such materials can also be used.

EP 2 163 267 B1

Examples of the release liner usable in the present invention include glassine paper, polyethylene, polypropylene, polyester, polyethylene terephthalate, polystyrene, aluminum film, foamed polyethylene film, foamed polypropylene film etc., or a laminate of members selected therefrom, or those mentioned above with silicone processing, emboss processing etc., and the like.

In the patch and patch preparation of the present invention, an adhesive layer having a certain level of thickness can be formed on a support. As a result, an effect of protecting a skin lesion and a wounded surface from impacts on the skin and the like can be improved, and the adhesive layer can contain a large amount of medicament and the like. Accordingly, in the patch and patch preparation of the present invention, the adhesive layer preferably has a thickness of not less than 40 $\mu$m, more preferably not less than 100 $\mu$m. On the other hand, the thickness of the adhesive layer should be up to around 400 $\mu$m, preferably 300 $\mu$m. When the thickness exceeds 300 $\mu$m, an applied patch and the like may be misaligned to allow protrusion of an adhesive component, or adhesive residue on the skin.

The patch of the present invention can be provided as a medical material or hygienic material in the form of a sheet-like patch, film-like patch, pad-like patch and the like, and can be used as an alternative of gauze in adhesive plaster, an alternative of non-woven fabric in wound-covering dressings and the like for the protection of a lesion and a wounded part of the skin. In addition, the patch preparation of the present invention contains a medicament in an adhesive layer formed on a support, and can be provided as a matrix-type patch preparation, a reservoir-type patch preparation, a patch-type transdermal preparation and the like.

The medicament to be used for the patch preparation of the present invention is preferably one that can be transdermally administered to mammals such as human and the like. Specific examples of the medicament include general anesthetic, topical anesthetic, narcotic, hypnotic, sedative, antiepileptic drug, antiparkinsonian drug, psychoneurotic drug, anti-vertiginous drug, autonomic drug, antispasmodic drug, skeleton muscle relaxant, anti-histamine drug, cardiac stimulant, antiarrhythmic, diuretic, hypotensive drug, vasoconstrictor, coronary vasodilator, peripheral vasodilator, hyperlipidemia drug, circulatory organ drug, anapnoic, antitussive expectorant, hormone drug, antipyretic analgesic antiphlogistic drug, external medication for mattery diseases, antipruritic drug, astringent drug, parasitic dermatic disease drug, hemostatic, gout treatment drug, diabetes drug, anti-malignant tumor drug, antibiotics, chemical therapy drug, quit smoking aid and the like. The content of the aforementioned drugs in the adhesive layer is not particularly limited as long as it is an effective amount capable of exhibiting efficacy upon transdermal absorption and the properties of the adhesive layer of the patch preparation of the present invention are not impaired, and is preferably 0.01 wt% - 60 wt%.

Examples

The present invention is explained in more detail in the following by referring to Examples, which are not to be construed as limitative.

The formulations of Examples and Comparative Examples of the present invention are shown in Table 1. In each Example and Comparative Example, carboxylated liquid polyisoprene (weight average molecular weight=25,000, number of carboxyl groups in a molecule=10) as a liquid rubber having a crosslinkable functional group in a molecule, isopropyl myristate as an organic liquid component, and an alicyclic hydrocarbon resin (softening point=100°C) as a tackifier were mixed, and 1,3-bis(N,N-diglycidyl aminomethyl)cyclohexane (TETRAD-C, manufactured by Mitsubishi Gas Chemical) as a crosslinking agent was added to give a liquid for an adhesive layer. The liquid was applied to a release liner (silicone-treated polyethylene terephthalate such that the thickness after drying was 200 $\mu$m for Examples other than Example 4, and 100 $\mu$m for Example 4, and dried. Then, a polyethylene terephthalate support was press-bonded to an adhesive face of an adhesive layer and the laminate was aged at 60°C for 3 days. The thickness of the adhesive layer of each test piece (width 30 mm × length 50 mm) of Examples and Comparative Examples was measured using a dial gauge defined in the Japanese Industrial Standards (JIS) B7503.

Table 1

| | amount added (wt%) | | | |
|---|---|---|---|---|
| | carboxylated liquid polyisoprene | isopropyl myristate | alicyclic hydrocarbon resin | TETRAD-C |
| Example 1 | 39.9 | 39.9 | 19.9 | 0.3 |
| Example 2 | 53.1 | 26.6 | 19.9 | 0.4 |
| Example 3 | 61.2 | 18.4 | 19.9 | 0.5 |
| Example 4 | 39.9 | 39.9 | 19.9 | 0.3 |
| Comparative Example 1 | 29.9 | 49.9 | 20.0 | 0.2 |

(continued)

| | amount added (wt%) | | | |
|---|---|---|---|---|
| | carboxylated liquid polyisoprene | isopropyl myristate | alicyclic hydrocarbon resin | TETRAD-C |
| Comparative Example 2 | 69.6 | 9.9 | 19.9 | 0.6 |
| Comparative Example 3 | 39.7 | 39.7 | 19.9 | 0.7 |
| Comparative Example 4 | 39.9 | 20.0 | 39.9 | 0.2 |

The gel fraction was determined as follows for each of the above-mentioned Examples and Comparative Examples. The adhesive layer (about 0.02 g) of each sample of the Examples and Comparative Examples was taken, and the weight ($W_1$) thereof was accurately measured. Then, the layer was immersed in toluene at 23°C for 7 days, and toluene-soluble components were extracted. The insoluble matter was taken out, dried at 120°C for 3 hr and the weight ($W_2$) thereof was measured. The gel fraction was determined by the following formula.

$$\text{gel fraction (wt\%)} = W_2/(W_1 \times A/B) \times 100$$

A: weight of carboxylated liquid polyisoprene and TETRAD-C
B: total weight of carboxylated liquid polyisoprene, isopropyl myristate, alicyclic hydrocarbon resin and TETRAD-C

Next, each patch of the Examples and Comparative Examples was evaluated for adhesiveness, ball tackiness, flexibility of adhesive layer, and adhesive residue. The evaluation method of each evaluation item is shown below.

(1) adhesiveness

In a thermostatic chamber (23°C, relative humidity 65%), each sample of Examples and Comparative Examples was punched out to give a test piece (length 50 mm×width 12 mm). In the thermostatic chamber, a release liner was detached from each test piece to expose the adhesive face. The adhesive face of the test piece was adhered to a bakelite plate by one reciprocation of a 2 kg rubber roller. After standing still for 30 min, the maximum stress during peeling (detachment rate 300 mm/min) at 180° was measured by a tension tester (Autograph AG-IS (manufactured by SHIMADZU Corporation)).

(2) flexibility of adhesive layer

In this test, liquids for adhesive layers having the compositions shown in the above-mentioned Table 1 were applied to release liners same as the above-mentioned such that the thickness after drying was 100 μm, dried in an oven at 100°C for 5 min and a release liner made of the same material was adhered to the adhesive face to give a sheet-like patch. The patch was cut into a rectangle (width 20 mm, length 40 mm) to give a sample. Then, the sample was densely wound from the long side with the long side as the central axis to give cylindrical or rod-like test pieces. Each of the obtained test pieces was set to a tension tester (Autograph AG-IS (manufactured by SHIMADZU Corporation)) under the conditions of 23°C, relative humidity 65%, chuck interval 20 mm (length of grasping part each 10 mm), and the tension force (N) was measured at a tension strength of 300 mm/min. A 100% modulus stress was determined from the obtained results, and the value was used as a flexibility index.

(3) ball tackiness

According to the Japanese Industrial Standards (JIS) Z0237, the following test was performed. In this test, the samples of Examples and Comparative Examples were punched out into width 50 mm×length 50 mm and used as the test pieces for convenience. To be precise, under the conditions of 23°C, relative humidity 65%, the test piece was set on a ball rolling apparatus at an inclination angle of 30° in such a manner that the adhesive face was exposed, and fixed to avoid difference in the level of surface between the test piece and the approach. Then, balls having different diameters were rolled on the test piece, and the diameter of the largest ball that stopped still thereon for 5 seconds or longer was taken as a ball tackiness of the test piece.

(4) adhesive residue

Each sample of the Examples and Comparative Examples was punched out into width 12 mm×length 50 mm and used as a test piece. A release liner was detached from the test piece to expose an adhesive face, which was adhered to the skin surface for 24 hr, and the state of the skin after removing the test piece was evaluated according to the following evaluation criteria.
evaluation criteria:

◎; no change from before adhesion, no stickiness
○; slight stickiness on the skin
△; partial transfer of adhesive onto the skin
×; adhesive residue on entire skin surface

The evaluation results of gel fraction of each Example and Comparative Example, and each item mentioned above are shown in Table 2.

Table 2

| sample | gel fraction | adhesiveness | flexibility (100% modulus) | ball tackiness value | adhesive residue |
|---|---|---|---|---|---|
| | (%) | (N/12 mm) | ($\times 10^{-2}$) | (mmφ) | |
| Example 1 | 65 | 1.05 | 1.82 | 18 | ○ |
| Example 2 | 68 | 0.87 | 1.93 | 17 | ◎ |
| Example 3 | 71 | 0.82 | 2.63 | 16 | ◎ |
| Example 4 | 65 | 0.56 | 1.82 | 15 | ◎ |
| Comparative Example 1 | 71 | 1.03 | 0.91 | 17 | × |
| Comparative Example 2 | 79 | 0.60 | 3.13 | 15 | ◎ |
| Comparative Example 3 | 98 | 0.29 | 12.58 | 9 | ◎ |
| Comparative Example 4 | 29 | 1.26 | 1.06 | 18 | × |

As is clear from Table 2, Example 1 to Example 4 having an adhesive layer having a gel fraction of 50 wt% - 75 wt%, which were prepared using compositions containing a liquid rubber having a crosslinkable functional group in a molecule in a proportion of 39.9 wt%, 53.1 wt%, 61.2 wt% and 39.9 wt%, respectively, relative to the total amount of the composition, showed suitable adhesiveness and flexibility, and good evaluation results of ball tackiness value and adhesive residue. In addition, Example 4 wherein an adhesive layer having a thickness of 1/2 of Example 1 was prepared using a composition having the same composition as in Example 1 showed a slight decrease in the adhesive force and ball tackiness value, and good evaluation of adhesive residue resulting from the decrease.
In contrast, Comparative Example 1 wherein the composition used for the preparation of an adhesive layer had a low content of the liquid rubber having a crosslinkable functional group in a molecule of 29.9 wt% and a high tackifier content of 49.9 wt% showed good adhesiveness, flexibility of the adhesive layer and ball tackiness value, but remarkable adhesive residue was observed on the skin. In addition, Comparative Example 2 wherein the composition used for the preparation of an adhesive layer had a content of 69.6 wt% of the liquid rubber having a crosslinkable functional group in a molecule, which was higher than the upper limit of the amount of the component in the present invention, and a gel fraction exceeding 75% showed lower flexibility of the adhesive layer. Moreover, Comparative Example 3 wherein the composition used for the preparation of an adhesive layer had a content of 39.7 wt% of the liquid rubber having a crosslinkable functional group in a molecule, which was within the range of the amount used for the adhesive layer of the patch of the present invention, but a high gel fraction of 98 wt% had a very hard adhesive layer, and remarkably low adhesiveness, as evidenced by the lower ball tackiness value. On the other hand, Comparative Example 4 wherein the gel fraction of the adhesive layer was less than 50 wt% showed a decrease in the cohesion strength of the adhesive layer, and the adhesive residue remained on the entire skin surface after removal of the layer.

As shown in Table 2, Example 1 to Example 4 of the present invention showed good flexibility. When the part applied with the patches of Example 1 - Example 4 was moved, lifting of the patch from the skin or development of wrinkles on the patch was not observed, thus demonstrating good followability to skin deformation.

From the foregoing results, the patches of the Examples of the present invention had an adhesive layer with a thickness of 100 $\mu$m - 200 $\mu$m, showed well-balanced, appropriate adhesiveness and flexibility as compared to the patches of the Comparative Examples, left small adhesive residue upon removal from the skin, and therefore, are suitable as patch.

**Claims**

1. A patch comprising a support and an adhesive layer formed on at least one surface of the support, wherein the adhesive layer has a gel fraction of 50 wt% - 75 wt%, and is comprised of a composition containing 33 wt% - 65 wt% of a liquid rubber having 3-20 crosslinkable functional groups in a molecule, wherein the composition is crosslinked using a crosslinking agent in 0.01 wt% to 4 wt% of the total amount of the composition used for preparation of the adhesive layer, and the gel fraction means the ratio of the weight of insoluble matter obtained by immersing an adhesive layer in toluene, to the components involved in the crosslinking of the adhesive layer.

2. The patch of claim 1, wherein the adhesive layer is comprised of a composition further containing 10 wt% - 40 wt% of an organic liquid component.

3. The patch of claim 1 or 2, wherein the liquid rubber is a liquid isoprene rubber having a crosslinkable functional group in a molecule.

4. The patch of claim 2 or 3, wherein the organic liquid component is one or more kinds selected from fats and oils, hydrocarbon, fatty acid ester and higher fatty acid.

5. The patch of claim 4, wherein the fatty acid ester is fatty acid monoalkyl ester.

6. The patch of claim 5, wherein the fatty acid monoalkyl ester is one or two kinds selected from isopropyl myristate and isopropyl palmitate.

7. The patch of any one of claims 1 to 6, wherein the adhesive layer is comprised of a composition further containing 15 wt% - 45 wt% of a tackifier.

8. The patch of any one of claims 1 to 7, wherein the adhesive layer has a thickness of not less than 40 $\mu$m.

9. A patch preparation comprising the patch of any one of claims 1 to 8, wherein the adhesive layer contains a medicament.

**Patentansprüche**

1. Pflaster, umfassend einen Träger und eine auf wenigstens einer Fläche des Trägers gebildete Klebeschicht, wobei die Klebeschicht eine Gelfraktion von 50 Gew.-% bis 75 Gew.-% aufweist und aus einer Zusammensetzung besteht, die 33 Gew.-% bis 65 Gew.-% eines flüssigen Kautschuks mit 3-20 vernetzungsfähigen funktionellen Gruppen in einem Molekül enthält, wobei die Zusammensetzung unter Verwendung eines Vernetzungsmittels in 0,01 Gew.-% bis 4 Gew.-% der Gesamtmenge der zur Herstellung der Klebeschicht verwendeten Zusammensetzung vernetzt ist, und "Gelfraktion" das Verhältnis des Gewichts der unlöslichen Substanz, die durch Eintauchen einer Klebeschicht in Toluol erhalten wird, zu den an der Vernetzung der Klebeschicht beteiligten Komponenten bedeutet.

2. Pflaster gemäß Anspruch 1, wobei die Klebeschicht aus einer Zusammensetzung besteht, die weiterhin 10 Gew.-% bis 40 Gew.-% einer organischen flüssigen Komponente enthält.

3. Pflaster gemäß Anspruch 1 oder 2, wobei der flüssige Kautschuk ein flüssiger Isoprenkautschuk ist, der eine vernetzungsfähige funktionelle Gruppe in einem Molekül aufweist.

4. Pflaster gemäß Anspruch 2 oder 3, wobei es sich bei der organischen flüssigen Komponente um eine oder mehrere Arten handelt, die aus Fetten und Ölen, Kohlenwasserstoff, Fettsäureester und höherer Fettsäure ausgewählt sind.

5. Pflaster gemäß Anspruch 4, wobei der Fettsäureester ein Fettsäuremonoalkylester ist.

6. Pflaster gemäß Anspruch 5, wobei es sich beim dem Fettsäuremonoalkylester um eine oder zwei Arten handelt, die aus Isopropylmyristat und Isopropylpalmitat ausgewählt sind.

7. Pflaster gemäß einem der Ansprüche 1 bis 6, wobei die Klebeschicht aus einer Zusammensetzung besteht, die weiterhin 15 Gew.-% bis 45 Gew.-% eines Klebrigmachers enthält.

8. Pflaster gemäß einem der Ansprüche 1 bis 7, wobei die Klebeschicht eine Dicke von nicht weniger als 40 $\mu$m aufweist.

9. Pflasterpräparat, umfassend das Pflaster gemäß einem der Ansprüche 1 bis 8, wobei die Klebeschicht ein Medikament enthält.

## Revendications

1. Patch comprenant un support et une couche adhésive formée sur au moins une surface du support, dans lequel la couche adhésive a une fraction de gel de 50 à 75 % en poids, et comprend une composition contenant un pourcentage massique de 33 à 65 % d'un caoutchouc liquide ayant de 3 à 20 groupes fonctionnels réticulables par molécule, dans lequel la composition est réticulée à l'aide d'un agent de réticulation en un pourcentage massique de 0,01 à 4 % de la quantité totale utilisée pour la préparation de la couche adhésive, et la fraction en gel désigne le rapport massique de matière insoluble obtenue par immersion d'une couche adhésive dans du toluène, par rapport aux composants impliqués dans la réticulation de la couche adhésive.

2. Patch selon la revendication 1, dans lequel la couche adhésive comprend une composition contenant en outre un pourcentage massique de 10 à 40 % d'un composant liquide organique.

3. Patch selon la revendication 1 ou 2, dans lequel le caoutchouc liquide est un caoutchouc isoprène liquide ayant un groupe fonctionnel réticulable dans une molécule.

4. Patch selon la revendication 2 ou 3, dans lequel le composant liquide organique est d'un ou de plusieurs types choisis parmi des graisses et des huiles, un hydrocarbure, un ester d'acide gras et un acide gras supérieur.

5. Patch selon la revendication 4, dans lequel l'ester d'acide gras est un ester monoalkylique d'acide gras.

6. Patch selon la revendication 5, dans lequel l'ester monoalkylique d'acide gras est d'un ou de deux types choisis parmi le myristate d'isopropyle et le palmitate d'isopropyle.

7. Patch selon l'une quelconque des revendications 1 à 6, dans lequel la couche adhésive comprend une composition contenant en outre un pourcentage massique de 15 à 45 % d'un agent à pouvoir collant.

8. Patch selon l'une quelconque des revendications 1 à 7, dans lequel la couche adhésive a une épaisseur qui n'est pas inférieure à 40 $\mu$m.

9. Préparation d'un patch comprenant le patch selon l'une quelconque des revendications 1 à 8, dans laquelle la couche adhésive contient un médicament.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 3022020 A

- JP 10151185 A